# EUROPEAN PATENT APPLICATION

(11) **EP 2 172 115 A1**
(43) Date of publication of application: **07.04.2010**
(21) Application number: 09171663.9
(22) Date of filing: 29.09.2009
(51) Int. Cl.: A23L 1/03, A21D 13/00, A23L 2/52, A23F 3/14, A61K 33/00, A61P 25/32

(54) **Composition for use in the reduction of blood alcohol content**

(30) Priority: 03.10.2008 IT FO20080021
(71) Applicant: Biffi, Ivan, 47100 Forli' (IT)
(72) Inventor: Biffi, Ivan, 47100 Forli' (IT)
(74) Representative: Bottero, Carlo

(57) **Abstract**

Composition for reducing the blood alcohol content of an individual who has consumed alcoholic beverages which comprises potassium bitartrate (also known as "cream of tartar") and sodium bicarbonate. Such composition can be administered as such or, preferably, by means of a food preparation that facilitates and renders more pleasing the intake thereof, for example by means of baked products (biscuits and the like) or chocolates or candies, or also in liquid form, for example by dissolution in a beverage (fruit juice, tea and the like).

## Description

The present invention relates to a composition suitable for obtaining a significant reduction in the blood alcohol content in subjects having an altered blood alcohol content following intake of alcoholic beverages.

The ethyl alcohol (ethanol) taken in through alcoholic beverages, even in moderate quantities, enters into circulation in the human body in an extremely short period of time and causes typical, well known symptoms to arise in a person, including indisposition. The quantity of ethanol ingested is traditionally measured with the parameter of the so-called blood alcohol content (or alcoholaemia, or more precisely, ethanolaemia).

However, the rate of disposal of ethanol from the body is not equally fast, as it may take even several hours to eliminate quantities of ethanol that are not particularly high or in any case to reduce the blood alcohol content within acceptable values.

As is well known, when ethanol is ingested in moderate doses, it is oxidized thanks to a physiological mechanism. An enzyme secreted by the liver, alcohol dehydrogenase, oxidizes the alcohol in the presence of a substance (cofactor) called NAD (nicotine amide dinucleotide) and transforms it into acetaldehyde, then into acetic acid. This reaction frees 7 calories per gram of alcohol, which are physiologically usable up to a total of 600-700 calories, i.e. half of the body's basal intake. The quantity of alcohol that can be oxidized in an hour per kilogram of weight is called the ethyl dehydrogenation coefficient and varies from 60 to 80mg. This quantity is modified neither by muscular work nor by cold. It may therefore be estimated that 100 or 120g (i.e. approximately one litre of 12% wine) is the tolerable quantity of alcohol that an adult weighing approximately 70 kg can consume at different times in the space of twenty-four hours. When it is absorbed in larger doses or in the case of an alcoholic or undernourished person, ethanol is metabolized according to another still little known mechanism and becomes dangerous to the body. In fact, this mechanism brings about an oxidization (i.e. destruction) of nucleic acids and amino acids originating from muscle mass. The product of this oxidization is hydrogen peroxide, which, under the influence of an enzyme, the catalase, in turn oxidizes the ethanol. This mechanism, which multiplies the speed of ethanol oxidization by five, provokes a loss of protein. The ethanol is rapidly absorbed by the digestive system (by the duodenum in particular) and then diffused into the blood and organic liquids.

The blood alcohol content increases rapidly after the ingestion of an alcoholic beverage. It reaches the maximum level in about 45 minutes and returns to zero in a time interval varying between 6 and 20 hours after having reached its peak. The quantity of alcohol ingested being equal, this content is lower when the stomach is full of food (up to one half), higher in the case of high-alcohol liquids (cognac, whisky, etc.), and is likewise higher and reached earlier in subjects who have undergone a gastrectomy (removal of the stomach). Under fasting conditions, the ingestion, in a brief space of time, of three quarters of a litre of wine causes the blood alcohol content to rise to a value ranging between 0.50 and 0.80g/L, while the ingestion of 2 litres causes it to reach 2 or 3g/L.

Most of the ethanol (around 90%) is metabolized within the body, whereas a small part is eliminated through urine, sweat and expired air. The ratio of ethanolaemia to alveolar air is relatively constant: 80mg of ethanol per 100mL of blood produce 35µg/100mL of ethanol in expired air. The blood alcohol concentration is expressed in g/L, so as to compare the content measured with the maximum limit established by the Highway Code (currently 0.5g/L).

Ethanol can be detected in human expiration thanks to the fact that as blood flows to the pulmonary alveoli it expels part of the alcohol contained in it through the breath, in a concentration which, as pointed out above, is proportional to the quantity of alcohol present at that moment in the blood itself. For this reason, by analyzing the quantity of ethanol expelled with breathing it is possible to derive the blood alcohol content.

The breath analyzer is based precisely on the above-illustrated principles, since by measuring the concentration of ethanol in expired air it can determine the blood alcohol content in the subject submitted to analysis. Prior to the introduction of breath analyzers, the blood alcohol content could be determined only by taking a sample of blood from the subject, thus an invasive measurement not performable in real time. It must be borne in mind that the measurement of blood alcohol content using a breath analyzer, despite being very precise, is not absolute, i.e. it varies from person to person depending on many personal factors, such as metabolism or quantity of fat.

A first type of breath analyzer is the so-called chemical breath analyzer, which consists of an inflatable bag (balloon) and a vial containing salts that react with the ethanol contained in the breath. This type of breath analyzer is easy to use: the bladder is inflated by releasing into it, through a special opening, the air expired by the subject; the previously uncapped vial is inserted into the opening and the bladder is deflated, thus causing the salts to come into contact with the breath. The value is read by comparing the colour taken on by the salts with that shown on a graduated scale printed on the vial.

The chemical reaction that occurs between the salts of the vial and the ethanol contained in the breath is of the oxido-reductive type. The reagents used in this reaction are potassium dichromate and sulphuric acid, which oxidize the ethanol contained in the bladder. The reaction products obtained are: chrome sulphate, potassium sulphate, acetic acid and water. The ethanol undergoes oxidation, becoming acetic acid, whereas the chrome is reduced from +6 to +3.

The characteristic that enables the quantity of alcohol to be measured is the colour change that the vial undergoes. This is due to the colour difference between reagents and products: potassium dichromate is yellow-orange in colour whereas chrome sulphate is green-blue in colour. Obviously, the more ethanol is present in the balloon, the more evident the chromatic variation in the vial will be.

Another type of breath analyzer in use is the so-called electronic breath analyzer. This consists of a machine body, a mouthpiece and a display. The functioning of an electronic breath analyzer, as opposed to a chemical one, does not use a salt, but rather relies on gas sensors that detect the presence of ethanol. The use of this device is nonetheless substantially identical to that of a chemical breath analyzer; the concept is always that of blowing into a container, though in this case one blows directly onto the sensor through the mouthpiece. Moreover, before making a measurement, it is necessary to wait a period of time in order for the sensor to warm up and work correctly. The value is read from the display, which shows the exact value.

The sensors used in the breath analyzer utilise a semiconductor as the sensitive detection element. The semiconductor usually used in breath analyzers is tin oxide. The sensors are composed on the inside of a small ceramic plate whereupon a sensitive element and a heating element are placed. The sensitive element consists of a thin layer of tiny granules of tin dioxide or of other oxides of transition elements like zinc, tungsten or molybdenum. The heating element is a normal resistor that serves exclusively to bring the sensor to the operating temperature. The semiconductor reacts with air, placing electrons in common and causing the formation of a potential barrier, i.e. a voltage that opposes the passage of electrons. Under normal conditions, for example with oxygen alone, this barrier is very high, but in the presence of a reducing gas, ethanol in our case, the oxygen reacting therewith does not react with the semiconductor and therefore a decrease in the barrier occurs. From this it may be deduced that the sensitive element has a resistance inversely proportional to the quantity of reducing gas; in other words, the more ethanol is present in the breath, the lower the potential barrier will be.

Various attempts have been made to seek to accelerate the disposal of ethanol by administering various substances, but without attaining positive results.

The Applicant has now surprisingly found that is possible to very significantly reduce the blood alcohol content of a subject who has consumed alcoholic beverages by administering to the subject him/herself a composition that comprises potassium bitartrate (also known as "cream of tartar") and sodium bicarbonate. Such composition can be administered as such or, preferably, by means of a food preparation that facilitates and renders more pleasing the intake thereof, for example by means of baked products (biscuits and the like) or chocolates or candies, or also in liquid form, for example by dissolution in a beverage (fruit juice, tea and the like).

Preferably, in the composition according to the present invention the weight ratio of potassium bitartrate to sodium bicarbonate ranges between 1:1 and 3:1, preferably between 1.9:1 and 2.1:1. Even more preferably, the two aforesaid components are used in a weight ratio of 2:1.

The intake of the composition according to the present invention by a subject who has previously ingested alcohol-containing beverages results in a more rapid process of ethanol disposal and a lowering of the blood alcohol content.

In the case in which the composition is employed in a baked product, it may be included within a common recipe, which entails the mixing, according to normal culinary techniques, of such basic ingredients as soft wheat flour, eggs, sugar and lard, to which the composition according to the invention is added. Corresponding substitutes can obviously also be used. The quantities can be indicated in the following proportion: for one kilogram of flour, 7 eggs, 250 grams of sugar and 150 grams of lard. The proportion and dosing of the basic ingredients should not be considered as rigorous, as they may be varied according to taste.

Following preparation of the mixture, the composition according to the invention, which performs the function of a leavening agent, is added in sufficient quantities to make the preparation rise, thus approximately a quantity greater than 2 grams per kilogram of flour.

The dough is then baked in an oven at medium temperature (around 200°C) for a time of around 20 minutes.

In the case of a chocolate, the latter typically has a weight of around 15-30 grams and contains 2-5 grams of the composition according to the invention.

The method of innovative use refers to the intake of the preparation through food in order to induce a more rapid process of ethanol disposal by the human body and a lowering of the blood alcohol content.

From this point of view, taking the composition according to the present invention in a sufficient quantity, which may vary based on individual physical characteristics and may be indicated, by way of example, as approximately 50 grams, induces and favours, in a subject who has previously ingested alcoholic beverages, a greater rapidity of the natural metabolic process of lowering the blood alcohol content.

Appreciable results may already be observed 15-20 minutes after intake of the preparation, although the response times and percentage decreases in the blood alcohol content may vary from subject to subject and are obviously proportional to the quantities of alcohol ingested.

The individual physical characteristics of the subject may obviously influence the result, with the best outcome being achieved in healthy subjects who are not habitual and heavy drinkers and for non-pathological quantities of alcohol.

The compositional and nutritional characteristics of the food preparation that includes the composition according to the invention, and which consists of a mixture of carbohydrates, protein, fats and leavening agents, render it usable without any particular subjective restrictions, except as dictated by pre-existing pathological conditions, such as diabetes, which contraindicate consumption of the foods in question.

The composition according to the present invention may also be added to other food preparations, e.g. chocolates or candies, or to beverages of varying nature, e.g. fruit juices, tea and the like. The choice of food preparation must naturally be made taking into account the final sensory result so as to guarantee an optimal appearance and taste for the consumer, in addition to enabling the food preparation to be packaged using easily achievable industrial and/or artisan techniques.

Alternatively, the composition according to the present invention can be administered in the form of tablet capsules, granulates, beads and the like, by the addition of suitable excipients and/or adjuvants known in the pharmaceutical sector, such as vehicles, dispersants, flavourings, sweeteners, stabilisers, preservatives, antioxidants and the like.

### EXAMPLE.

Biscuits were made according to a conventional recipe using a mixture made up of potassium bitartrate and sodium bicarbonate in a weight ratio of 2:1 as the leavening agent, in a quantity equal to 90 grams per kg of dough.

Chocolates were likewise made according to a conventional recipe, to which was added a mixture made up of potassium bitartrate and sodium bicarbonate in a weight ratio of 2:1, in a quantity equal to 130 grams per kg of chocolates.

Using an electronic breath analyzer, the blood alcohol content was measured in a 40-year-old woman weighing approximately 80 kg, immediately after the administration of wine and grappa (time zero) and after specific intervals of time as shown below.

In Test 1 (benchmark), after consuming the alcoholic beverages the woman did not ingest any other substances; in Test 2, immediately after consuming the alcoholic beverages (time zero) the subject ingested a biscuit prepared as described above (weight equal to about 40-50g), in Test 3, three chocolates prepared as described above, each weighing about 16g.

The results of the blood alcohol content measurements (expressed as mg/L) are shown in the following Table 1.

**TABLE 1**

| Time (min) | 1 | 2 | 3 |
|---|---|---|---|
| 0 | 1.55 | 0.84 | 0.84 |
| 10 | 0.33 | 0.24 | 0.28 |
| 20 | 0.47 | -- | -- |
| 25 | -- | 0.25 | -- |
| 30 | 0.53 | -- | -- |
| 40 | 0.69 | 0.27 | 0.22 |
| 50 | 0.68 | -- | -- |
| 55 | -- | 0.24 | -- |
| 60 | 0.68 | -- | -- |
| 70 | -- | -- | 0.16 |
| 90 | -- | -- | 0.00 |

From the results shown above, it is clearly evident that a reduction in blood alcohol content is obtained following intake of a food preparation according to the present invention.

## Claims

1. A composition comprising potassium bitartrate and sodium bicarbonate for use in the reduction of blood alcohol content.

2. The composition according to claim 1, wherein the weight ratio of potassium bitartrate to sodium bicarbonate ranges between 1:1 and 3:1, preferably between 1.9:1 and 2.1:1.

3. The composition according to claim 2, wherein the weight ratio of potassium bitartrate to sodium bicarbonate is equal to 2:1.

4. A food preparation comprising a composition according to any of the claims 1 to 3 for use in the reduction of blood alcohol content.

5. The food preparation according to claim 4 in the form of a baked product.

6. The food preparation according to claim 4 in the form of a chocolate or candy.

7. The food preparation according to claim 4 in the form of a beverage.

8. A formulation comprising a composition according to any of the claims 1 to 3 in the form of a tablet, capsule, granulate or bead.

9. The use of a composition according to any of the claims 1 to 3, in the reduction of blood alcohol content.

10. The use of a food preparation according to any of the claims 4 to 7, in the reduction of blood alcohol content.

11. The use of a formulation according to claim 8, in the reduction of blood alcohol content.
